# EUROPEAN PATENT SPECIFICATION

(11) **EP 2 812 696 B1**
(45) Date of publication and mention of the grant of the patent: **14.11.2018**
(21) Application number: 13746745.2
(22) Date of filing: 08.02.2013
(51) Int. Cl.: G01N 33/53, G01N 33/543, G01N 33/564

(54) **METHODS FOR MEASURING HIGH MOLECULAR WEIGHT COMPLEXES OF FIBRINOGEN WITH FIBRONECTIN AND FIBULIN-1**
VERFAHREN ZUR MESSUNG HOCHMOLEKULARER KOMPLEXE VON FIBRINOGEN MIT FIBRONECTIN UND FIBULIN-1
PROCÉDÉ DE MESURE DE COMPLEXES DE POIDS MOLÉCULAIRE ÉLEVÉ FORMÉS À PARTIR DU FIBRINOGÈNE AVEC DE LA FIBRONECTINE ET DE LA FIBULINE-1

(30) Priority: 10.02.2012 US 201213370757
(43) Date of publication of application: 17.12.2014
(73) Proprietor: MSDX, Inc., Tucson, AZ 85747 (US)
(72) Inventor: NAYAK, Ramesh, C., Tucson, AZ 85704 (US)
(74) Representative: Held, Stephan
(86) International application number: PCT/US2013/025388
(87) International publication number: WO 2013/119986

(56) References cited:
- WO-A1-2010/005387
- WO-A1-2011/127587
- US-A- 4 703 004
- US-A1- 2010 215 634
- R. NAYAK ET AL: "a circulating biomarker for therapeutic response in multiple sclerosis", 5TH JOINT TRIENNIAL CONGRESS OF THE EUROPEAN AND AMERICAS COMMITTEES FOR TREATMENT AND RESEARCH IN MULTIPLE SCLEROSIS AMSTERDAM, THE NETHERLANDS, 21 October 2011 (2011-10-21), page ABST., XP055078424,
- THE BIOMS STUDY GROUP ET AL: "Cerebrospinal fluid biomarkers in multiple sclerosis", NEUROBIOLOGY OF DISEASE, BLACKWELL SCIENTIFIC PUBLICATIONS, OXFORD, GB, vol. 35, no. 2, 1 August 2009 (2009-08-01) , pages 117-127, XP026932754, ISSN: 0969-9961, DOI: 10.1016/J.NBD.2009.04.010 [retrieved on 2009-05-05]
- NAYAK ET AL.: 'A Circulating Biomarker for Therapeutic Response in Multiple Sclerosis.' THE 5TH JOINT TRIENNIAL CONGRESS OF THE EUROPEAN AND AMERICAS COMMITTEES ON TREATMENT AND RESEARCH IN MULTIPLE SCLEROSIS (ECTRIMS AND ACTRIMS), [Online] 21 October 2011, XP055078424 Retrieved from the Internet: <URL:http://www.google.coMurl?sa=t8rct=j8q= 8esrc=s8source=web8cd=2&ved=OCDUQFjAB&url=h ttp%3A%2F%2Fwww.msdx.co%2Fassets%2Fdocs%2F2 011 %2520ECTRIMS%2520Conferenc e%2520- %2520MS%2520Biomarl<er.pdf&ei=dxKTUbmuEYSDi AK3ooGQDQ&usg=AFQJCNGsng1 fRwyX 58eWBu4TLCedFCAPBg8sig2=39ZS-nW3drXPUFt9CCP HagBbvm=bv.4647102> [retrieved on 2013-05-15]
- BALBONA ET AL.: 'Fibulin binds to itself and to the carboxyl-terminal heparin-binding region of fibronectin.' J. BIOL. CHEM. vol. 267, no. 28, 05 October 1992, pages 20120 - 20125, XP055078426
- OTTERVALD ET AL.: 'Multiple Sclerosis: Identification and Clinical Evaluation of Novel CSF Biomarkers' J. PROTEOMICS vol. 73, 2010, pages 1117 - 1132, XP027098857

## Description

### BACKGROUND OF THE INVENTION

R. Nayak et al. ("a circulating biomarker for therapeutic response in multiple sclerosis" , 5th Joint triennial congress of the European and Americas Committees for Treatment and Research in Multiple Sclerosis Amsterdam, The Netherlands, 21 October 2011 (2011-12-21), page ABST) discloses a circulating high molecular weight protein complex that binds a small peptide (21 aa). The protein complex is associated with multiple sclerosis relative to healthy controls. The marker is attenuated by some disease modifying therapies and may be a means of monitoring disease activity and therapeutic response to disease modifying therapies in multiple sclerosis.
It has been surprisingly discovered that the expression of a protein complex (e.g. an aggregate, a complex) termed "MSDX Complex-1" is elevated in multiple sclerosis patients as compared to healthy controls. MSDX Complex-1 is a high molecule weight complex comprising fibrinogen, fibronectin, and fibulin-1. MSDX Complex-1 alone or in combination with other markers may be useful as an indicator of multiple sclerosis or other diseases or conditions, for example for an inflammatory condition, a neurodegenerative disease or condition, cancer, stroke, or other diseases. MSDx complex-1 alone or in combination with one or more other biomarkers may help monitor disease activity (e.g., relapse, remission, etc.). Monitoring disease activity may be useful for detecting a response (e.g., positive response, negative response, lack of response) to a therapy, for detecting patient compliance with a therapy, or for providing useful clinical information for disease management.

The present disclosure features methods for measuring high molecular weight complexes of fibrinogen with fibronectin and fibulin-1 ("MSDx Complex-1") and applications thereof. The methods may be used to monitor disease activity and therapeutic efficacy in diseases or conditions that have an inflammatory component, for example autoimmune diseases, neurodegenerative diseases, cancers and metabolic diseases such as type 2 diabetes mellitus. The present disclosure is not limited to the aforementioned diseases and conditions or the aforementioned applications.

### SUMMARY OF THE INVENTION

The present disclosure features the use of MSDX Complex-1 for detection and monitoring of various diseases (and disease activity), for example for detection and/or monitoring of multiple sclerosis and/or other inflammatory, autoimmune or neurodegenerative diseases.

For example, the present disclosure features methods of detecting MSDX Complex-1. In some aspects, the method comprises introducing a first antibody to a sample to create an antibody-sample mixture, wherein the first antibody is specific for one of fibrinogen, fibronectin, or fibulin-1. The first antibody comprises a label molecule. The method may further comprise providing a well coated with a second antibody, wherein the second antibody is specific for one of fibrinogen, fibronectin, or fibulin-1, introducing the antibody-sample mixture to the well; and introducing a substrate to the antibody-sample mixture in the well, wherein the label molecule and the substrate interact to provide a signal. In some aspects, when the signal is detected then MSDX Complex-1 is detected.

In some aspects, the sample comprises blood. In some aspects, the sample comprises serum, plasma, cerebrospinal fluid (CSF), synovial fluid, cystic fluid, lymph fluid, ascites, pleural effusion, interstitial fluid, ocular fluids, vitreal fluid, or a combination thereof. In some aspects, the first antibody is an anti-fibulin-1 antibody and the second antibody is an anti-fibrinogen antibody. In some aspects, the first antibody is an anti-fibronectin antibody and the second antibody is an anti-fibrinogen antibody. In some aspects, the first antibody is an anti-fibrinogen antibody and the second antibody is an anti-fibrinogen antibody. In some aspects, the first antibody is an anti-fibulin-1 antibody and the second antibody is an anti-fibronectin antibody. In some aspects, the first antibody is an anti-fibronectin antibody and the second antibody is an anti-fibronectin antibody. In some aspects, the first antibody is an anti-fibrinogen antibody and the second antibody is an anti-fibronectin antibody. In some aspects, the first antibody is an anti-fibulin-1 antibody and the second antibody is an anti-fibulin-1 antibody. In some aspects, the first antibody is an anti-fibronectin antibody and the second antibody is an anti-fibulin-1 antibody. In some aspects, the first antibody is an anti-fibrinogen antibody and the second antibody is an anti-fibulin-1 antibody.

In some aspects, the method further comprises introducing a third antibody to the antibody-sample mixture prior to introduction to the well, wherein the third antibody is specific for one of fibulin-1, fibronectin, or fibrinogen, wherein the third antibody has a different specificity than the first antibody. In some aspects, the method further comprises introducing a third antibody to the antibody-sample mixture prior to introduction to the well, the third antibody is specific for one of fibulin-1, fibronectin, or fibrinogen, wherein the third antibody has a different specificity than the first antibody and a different specificity than the second antibody.

In some aspects, the label molecule comprises an enzyme. In some aspects, the enzyme comprises horseradish peroxidase. In some aspects, the signal is a chemiluminescent signal, a fluorescent signal, a colorimetric signal, a potentiometric signal, an amperometric signal, or a combination thereof.

The present invention features a method of detecting MSDX Complex-1 in a sample, MSDX Complex-1 being a protein complex comprising fibrinogen, fibronectin, and fibulin-1. The method comprises introducing a labeled peptide and an anti-peptide antibody to the sample to create an antibody-sample mixture, wherein MSDX Complex-1 competes with the anti-peptide antibody for binding to the labeled peptide; providing a well coated with a well antibody; introducing the antibody-sample mixture to the well; introducing a substrate to the well, wherein a label molecule of the labeled peptide and the substrate interact to provide a level of a signal; wherein a level of signal is indicative of a level of MSDX Complex-1 in the sample. Further disclosed is comparing the level of the signal to a control, wherein if the level of the signal is higher than that of the control then MSDX Complex-1 is lower than that in the control, wherein if the level of the signal is lower than that of the control then MSDX Complex-1 is higher than that in the control.

In some embodiments, the label molecule is biotin. In some embodiments, the labeled peptide comprises at least SEQ ID NO: 1. In some embodiments, the labeled peptide comprises at least SEQ ID NO: 2. In some embodiments, the labeled peptide comprises at least SEQ ID NO: 3. In some embodiments, the label molecule of the labeled peptide is located at the C-terminus, the N-terminus or at both termini. In some embodiments, the labeled peptide is between about 13 to 50 amino acids in length. In some embodiments, the labeled peptide has a pi of about 6.1. In some embodiments, the labeled peptide has a net charge of about -0.1 at pH 7.0. In some embodiments, the labeled peptide comprises an epitope tag disposed at the C-terminus, the N-terminus, or at both termini.

Disclosed is a method of detecting a disease or condition in a patient, wherein the method comprises obtaining from a patient a fluid sample from outside of a brain tissue of the patient; and detecting MSDX Complex-1 in the fluid sample, wherein MSDX Complex-1 is a protein complex comprising fibrinogen, fibronectin, and fibulin-1.

In some embodiments, the sample comprises blood. In some embodiments, the sample comprises cerebrospinal fluid (CSF), synovial fluid, cystic fluid, lymph fluid, ascites, pleural effusion, interstitial fluid, ocular fluids, vitreal fluid, urine, or a combination thereof. In some aspects, the condition is an autoimmune disease, a neurodegenerative disease, a metabolic disease, a neurological condition, an inflammatory condition, or a cancer. In some aspects, the condition is multiple sclerosis.

Disclosed is a method of monitoring a disease or condition or monitoring a therapy. In some aspects, the method comprises obtaining from a patient a fluid sample from outside of a brain tissue of the patient, the fluid sample is obtained at time T0; detecting a level of MSDX Complex-1 in the fluid sample; obtaining from a patient a fluid sample from outside of a brain tissue of the patient, the fluid sample is obtained at time T1, wherein T1 is after T0; comparing the level of MSDX Complex-1 in the fluid sample from T0 to the level of MSDX Complex-1 in the fluid sample from T1.

The present disclosure also features MSDX Complex-1 for use in detecting a disease (or for monitoring a disease) according to the methods of the present invention. For example, in some aspects, the present disclosure features MSDX Complex-1 for use in a method of detecting a disease, wherein the method comprises introducing a first antibody to a sample to create an antibody-sample mixture, wherein the first antibody is specific for one of fibrinogen, fibronectin, or fibulin-1. The first antibody comprises a label molecule. The method may further comprise providing a well coated with a second antibody, wherein the second antibody is specific for one of fibrinogen, fibronectin, or fibulin-1, introducing the antibody-sample mixture to the well; and introducing a substrate to the antibody-sample mixture in the well, wherein the label molecule and the substrate interact to provide a signal. In some aspects, when the signal is detected then MSDX Complex-1 is detected.

In some aspects, the present disclosure features MSDX Complex-1 for use in a method of detecting a disease, wherein the method comprises obtaining from a patient a fluid sample from outside of a brain tissue of the patient, the fluid sample is obtained at time T0; detecting a level of MSDX Complex-1 in the fluid sample; obtaining from a patient a fluid sample from outside of a brain tissue of the patient, the fluid sample is obtained at time T1, wherein T1 is after T0; comparing the level of MSDX Complex-1 in the fluid sample from T0 to the level of MSDX Complex-1 in the fluid sample from T1.

In some aspects, the present disclosure features MSDX Complex-1 for use in a method of detecting a disease, wherein the method comprises introducing a labeled peptide and an anti-peptide antibody to a sample to create an antibody-sample mixture, the anti-peptide antibody can bind to the labeled peptide, the labeled peptide comprises a label molecule; providing a well coated with a well antibody, the well antibody is specific for an anti-peptide antibody; introducing the antibody-sample mixture to the well; introducing a substrate to the antibody-sample mixture in the well, wherein the label molecule of the labeled peptide and the substrate interact to provide a level of a signal; and comparing the level of the signal to a control, wherein if the level of the signal is higher than that of the control then MSDX Complex-1 is lower than that in the control, wherein if the level of the signal is lower than that of the control then MSDX Complex-1 is higher than that in the control.

Diseases or conditions may include but are not limited to multiple sclerosis and/or other inflammatory, autoimmune or neurodegenerative diseases.

### BRIEF DESCRIPTION OF THE DRAWINGS

FIG. 1 shows the non-limiting example of a method of detecting MSDX Complex-1 described in EXAMPLE 1.
FIG. 2A and FIG. 2B show the non-limiting example of a method of detecting MSDX Complex-1 described in EXAMPLE 2.
FIG. 3 shows the non-limiting example of a method of detecting MSDX Complex-1 described in EXAMPLE 3.
FIG. 4 shows the non-limiting example of a method of detecting MSDX Complex-1 described in EXAMPLE 3.

### DESCRIPTION OF PREFERRED EMBODIMENTS

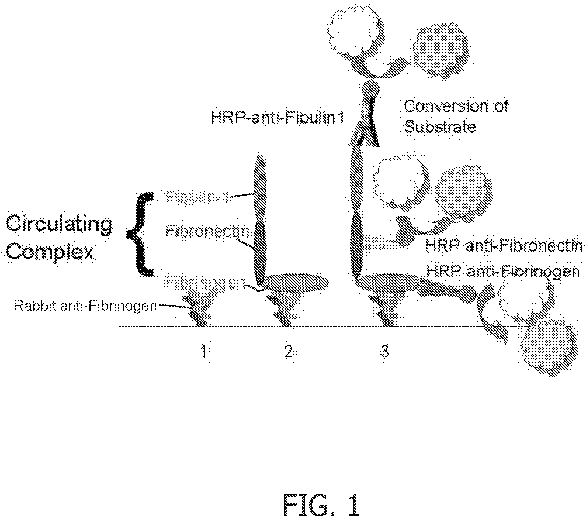

Referring now to FIG. 1-4, the present invention features methods for measuring high molecular weight complexes of MSDX Complex-1, e.g., fibrinogen with fibronectin and fibulin-1, in a sample. As used herein, the term "MSDx Complex-1" refers to a high molecular weight complex of fibrinogen, fibronectin, and fibulin-1. The detection of MSDX Complex-1 may be used for a variety of purposes, for example for detecting a disease or condition, for monitoring a disease or condition, for monitoring a therapy, etc.

A circulating high molecular weight protein complex has been found to bind certain small peptides selectively. For example, by sephacryl S200 gel filtration chromatography, the binding activity was found in a broad peak of 400,000-900,000 kD. This peak was collected and shown by LC/MS, after in solution protease digestion, to consist of Fibrinogen, Fibronectin and Fibulin-1. The present invention features a unique competitive ELISA assay format to measure the amount of MSDx Complex-1 in a sample, e.g., plasma, by its ability to compete with an anti-peptide antibody for binding of the labeled peptide (e.g., biotinylated peptide). In some embodiments, the method comprises introducing a labeled peptide and an anti-peptide antibody to a sample to create an antibody-sample mixture. The anti-peptide antibody can bind to at least the labeled peptide and MSDX Complex-1. The labeled peptide comprises a label molecule (e.g., biotin). The label molecule is not limited to biotin but may include any appropriate label. Labels are well known to one of ordinary skill in the art.

In some embodiments, the method further comprises providing a well (e.g., ELISA well) coated with a "well antibody". The well antibody is specific for an anti-peptide antibody. The method further comprises introducing the antibody-sample mixture to the well and introducing a substrate to the antibody-sample mixture in the well. The label molecule of the labeled peptide and the substrate interact to provide a signal. The level of the signal is compared to a control. If the level of the signal is higher than that of the control, then MSDX Complex-1 is not detected. If the level of the signal is lower than that of the control then MSDX Complex-1 is detected.

In some embodiments, the labeled peptide is or comprises SEQ ID NO: 1 (see EXAMPLE 2). In some embodiments, the labeled peptide is or comprises SEQ ID NO: 2 (see EXAMPLE 2). In some embodiments, the labeled peptide is or comprises SEQ ID NO: 3 (see EXAMPLE 2).

In some embodiments, the label of the labeled peptide is located at the C-terminus, the N-terminus or at both termini. In some embodiments, the labeled peptide is between about 15 to 50 amino acids in length, e.g., 24 amino acids, between about 15 to 40 amino acids, between about 15 to 30 amino acids, between about 20 to 30 amino acids, etc. In some embodiments, the labeled peptide has a pi of about 6.1. In some embodiments, the labeled peptide has a pi between about 6 and 7.0, between about 5.5 and 6.5, between about 5.8 and 6.4, etc. In some embodiments, the labeled peptide has a net charge of about -0.1 at pH 7.0. In some embodiments, the labeled peptide comprises an epitope tag disposed at the C-terminus, the N-terminus, or at both termini.

The present disclosure also features a method of detecting MSDX Complex-1 comprising introducing a first antibody to a sample to create an antibody-sample mixture, wherein the first antibody is specific for one of fibrinogen, fibronectin, or fibulin-1. The first antibody comprises a label molecule (e.g., HRP). A well (e.g., ELISA well) is provided. The well is coated with a second antibody, wherein the second antibody is specific for one of fibrinogen, fibronectin, or fibulin-1. In some aspects, the method further comprises introducing the antibody-sample mixture to the well and introducing a substrate to the antibody-sample mixture in the well. The label molecule and the substrate interact to provide a signal (e.g., a chemiluminescent signal, a fluorescent signal, a colorimetric signal, a potentiometric signal, an amperometric signal, or a combination thereof). When the signal is detected then MSDX Complex-1 is detected.

In some aspects, the first antibody is an anti-fibulin-1 antibody and the second antibody is an anti-fibrinogen antibody. In some aspects, the first antibody is an anti-fibronectin antibody and the second antibody is an anti-fibrinogen antibody. In some aspects, the first antibody is an anti-fibrinogen antibody and the second antibody is an anti-fibrinogen antibody. In some aspects, the first antibody is an anti-fibulin-1 antibody and the second antibody is an anti-fibronectin antibody. In some aspects, the first antibody is an anti-fibronectin antibody and the second antibody is an anti-fibronectin antibody. In some aspects, the first antibody is an anti-fibrinogen antibody and the second antibody is an anti-fibronectin antibody. In some aspects, the first antibody is an anti-fibulin-1 antibody and the second antibody is an anti-fibulin-1 antibody. In some aspects, the first antibody is an anti-fibronectin antibody and the second antibody is an anti-fibulin-1 antibody. In some aspects, the first antibody is an anti-fibrinogen antibody and the second antibody is an anti-fibulin-1 antibody.

In some aspects, the method further comprises introducing a third antibody to the antibody-sample mixture prior to introduction to the well, the third antibody is specific for one of fibulin-1, fibronectin, or fibrinogen, wherein the third antibody has a different specificity than the first antibody. In some aspects, the method further comprises introducing a third antibody to the antibody-sample mixture prior to introduction to the well, the third antibody is specific for one of fibulin-1, fibronectin, or fibrinogen, wherein the third antibody has a different specificity than the first antibody and a different specificity than the third antibody.

In some aspects, the label molecule comprises an enzyme. In some aspects, the enzyme comprises horseradish peroxidase.

In some aspects, the first antibody is a rabbit antibody. The first antibody is not limited to rabbit and may be any other appropriate antibody (e.g., mouse, human, etc.). In some aspects, the second antibody comprises an anti-rabbit antibody, e.g., a goat anti-rabbit antibody, a mouse anti-rabbit antibody, a human anti-rabbit antibody, etc.

In some aspects, the method of detection comprises attaching the peptide to a conventional bead or a magnetic bead, letting the complex bind to the peptide on the bead, and then collecting the beads and removing the unbound plasma proteins by washing. The bound material can be detected with the aid of antibodies to Fibrinogen B, Fibronectin or Fibulin-1.

As used herein, the term "about" refers to plus or minus 10% of the referenced number.

### EXAMPLE 1 not according to the invention

The following example describes an example of a method of detecting MSDX Complex-1 according to FIG. 1. Anti-Fibrinogen antibodies are immobilized onto an assay surface (e.g., ELISA well, glass slide, magnetic particle, antibody array matrix) and blocked using conventional methods. A biological fluid (e.g., serum, plasma, cerebrospinal fluid) is then contacted with the immobilized antibody and unbound material is washed off. Then antibodies to fibronectin and/or Fibulin-1 are contacted with the immobilized material and unbound antibodies are washed off. The bound antibodies are then detected with a labelled anti-immunoglobulin of the appropriate specificity to generate a measurable signal (the signal may be chemiluminescent, fluorescent, colorimetric, potentiometric, amperometric etc).

### EXAMPLE 2

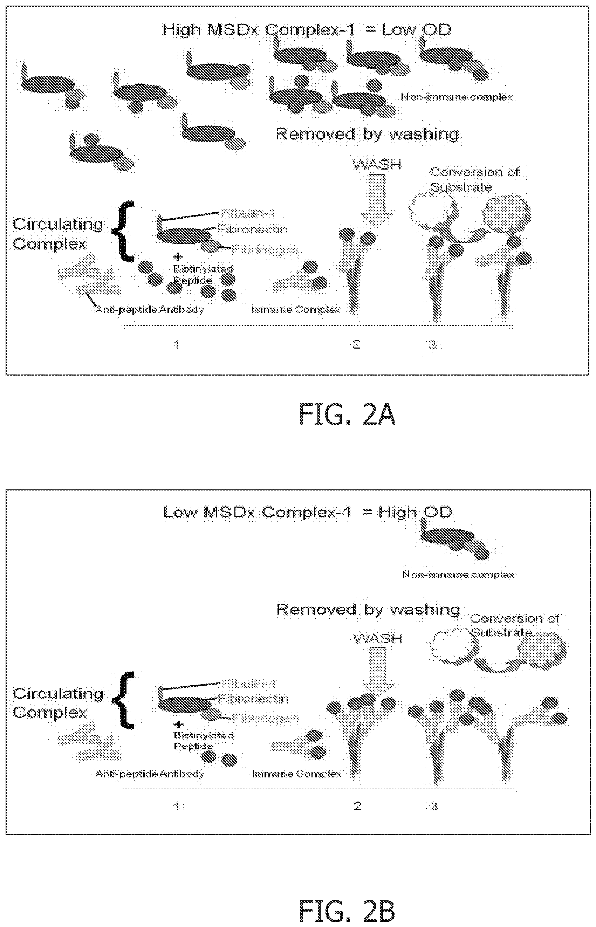

The following example describes an example of a method of detecting MSDX Complex-1 according to FIG. 2A and FIG. 2B. In some embodiments, method is a competitive ELISA assay format. In some embodiments, the competitive ELISA assay used for detecting MDSX Complex-1 utilizes a labeled analyte and measures the ability of an unlabelled native analyte in a biological fluid to compete with the labeled analyte for binding to the antibody. In this assay the labeled analyte is bound by an unrelated binding protein that prevents it's binding to antibody and is washed off before the detection step. A standard curve to quantify binding by MDSX Complex-1 is generated by competition with "cold" peptide.

ELISA wells coated with goat anti-rabbit IgG(Fc) trap immune complexes formed between a rabbit anti-peptide antibody and biotinylated peptide. MSDX Complex-1 in added plasma competes with the rabbit anti-peptide antibody for binding to biotinylated peptide. The more MSDX Complex-1 that is present in the plasma the more biotinylated peptide it binds leaving less available to bind to antibody. Thus high levels of MSDX Complex-1 result in low optical density and vice versa.

In some embodiments, the peptide is labeled at the C-terminal with biotin or another detection agent. In some embodiments, the N-terminal of the peptide may be amine or amide. In some embodiments, the peptide is 24 amino acids long. In some embodiments, the peptide sequence is: CQYRCFQVITNGIGLNLFKDPVAD (SEQ ID NO: 1). In some embodiments, the peptide has a pI of 6.1. In some embodiments, the peptide has a net charge of -0.1 at pH 7.0. In some embodiments, the peptide has an average hydrophilicity (Hopp & Woods method) of -0.3. In some embodiments, the peptide has a ratio of hydrophilic residues to total residues of 33%. In some embodiments, an epitope tag is attached to a terminus, e.g., the N-terminus, to enable the use of other capture antibodies, for example a polyHistidine tag (HisTag).

In some embodiments, any peptide sequence derived by conservative amino acid substitution rules such as the Dayhoff matrix and the like of SEQ ID NO: 1 may be used. In some embodiments, alternative peptides may be used.

In some embodiments, the peptide sequence is CSFKCYSVVTNGLGINVFKDPVAD (SEQ ID NO: 2). In some embodiments, the peptide has a pi of 6.1. In some embodiments, the peptide has a net charge of -0.1 at pH 7.0. In some embodiments, the peptide has an average hydrophilicity (Hopp & Woods method) of -0.3. In some embodiments, the peptide has a ratio of hydrophilic residues to total residues of 33%.

In some embodiments, the peptide sequence is CQYRCFQIITNGIGLNLFKDPVAD (SEQ ID NO: 3). In some embodiments, the peptide has a pi of 6.1. In some embodiments, the peptide has a net charge of -0.1 at pH 7.0. In some embodiments, the peptide has an average hydrophilicity (Hopp & Woods method) of -0.3. In some embodiments, the peptide has a ratio of hydrophilic residues to total residues of 33%.

The various peptides described bind selectively to a macromolecular complex consisting of Fibrinogen B, Fibronectin and Fibulin 1. The levels of this complex have surprisingly been found to be associated with neuroinflammatory diseases including multiple sclerosis. Addition of the peptide to plasma or serum causes the peptide to bind to the complex of Fibrinogen B, Fibronectin and Fibulin 1, affecting a transformation of matter that result in the formation of the aggrefatin complex.

### EXAMPLE 3

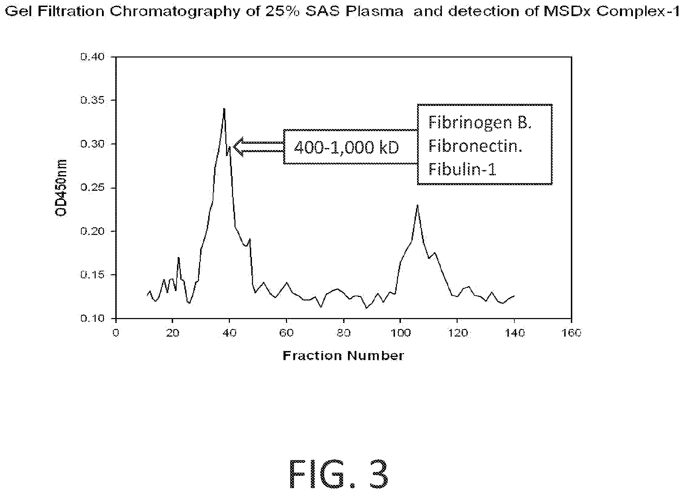

The following example describes an example of a method of detecting MSDX Complex-1 according to FIG. 3. By gel filtration chromatography, MSDx Complex-1 was seen to have a molecular weight range of 400,000 to 1,000,000 daltons.

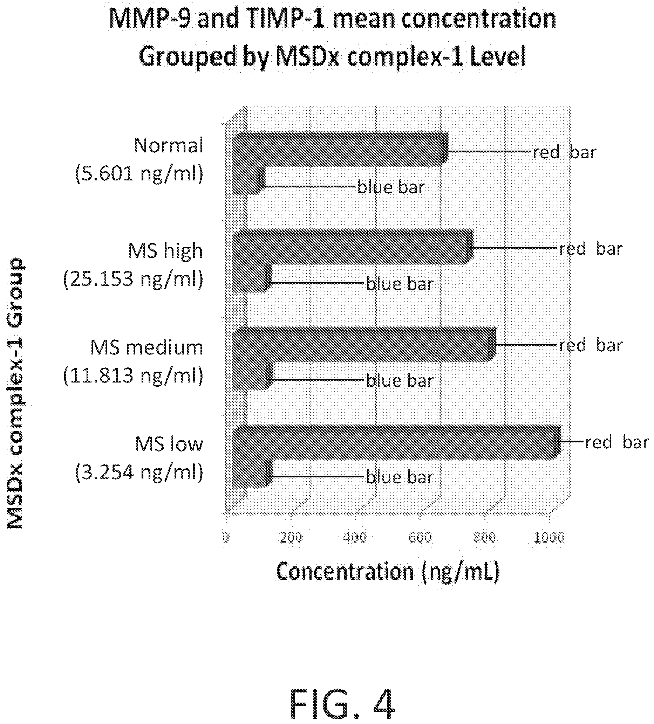

The following example describes an example of a method of detecting MSDX Complex-1 according to FIG. 4. This high molecular weight component of plasma was found to comprise of Fibrinogen-B, Fibronectin and Fibulin-1. As Fibronectin and Fibulin-1 are extracellular matrix proteins. Without wishing to limit the invention to any particular theory or mechanism, it is believed that these proteins are released from vascular basement membranes by proteolytic activity of leukocytes attempting to enter the brain from the blood circulation. Thus, the released proteins may then associate with Fibrinogen B to form large complexes. If this complex is generated as a consequence of basement membrane degradation by proteolysis, then the matrix metaloprotease MMP-9 is a likely candidate to participate in the generation of MSDx complex-1 since the MMP-9 levels (blue bars) were elevated in MS patients relative to normal subjects (p= 0.01 - 0.006). MMP-9 did not vary by MSDx complex-1 levels in MS subjects (p= not significant). The TIMP-1 levels (red bars) were high in subjects with low MSDx complex-1 levels, which is consistent with low proteolytic activity and potentially lower levels of leukocyte invasion and disease activity. MS subjects with high MSDX complex-1 levels had lower levels of TIMP-1, which is consistent with higher proteolytic activity and potentially higher levels of leukocyte invasion and disease activity (TIMP-1 in MSDx complex-1 high vs. Low p= 0.0033).

Various modifications of the invention, in addition to those described herein, will be apparent to those skilled in the art from the foregoing description. Such modifications are also intended to fall within the scope of the appended claims.

Although there has been shown and described the preferred embodiment of the present invention, it will be readily apparent to those skilled in the art that modifications may be made thereto which do not exceed the scope of the appended claims. Therefore, the scope of the invention is only to be limited by the following claims.

## Claims

1. A method of detecting MSDX Complex-1, MSDX Complex-1 being a protein complex comprising fibrinogen, fibronectin, and fibulin-1 in a sample, the method comprises:
(a) introducing to the sample an anti-peptide antibody and a labeled peptide to create an antibody-sample mixture, wherein MSDX Complex-1 competes with the anti-peptide antibody for binding to the labeled peptide;
(b) providing a well coated with a well antibody;
(c) introducing the antibody-sample mixture to the well; and
(d) introducing a substrate to the well, wherein a label molecule of the labeled peptide and the substrate interact to provide a level of a signal; wherein a level of signal is indicative of a level of_MSDX Complex-1 in the sample.

2. The method of claim 1, wherein the label molecule is biotin.

3. The method of claim 1, wherein the labeled peptide comprises at least SEQ ID NO: 1.

4. The method of claim 1, wherein the labeled peptide comprises at least SEQ ID NO: 2.

5. The method of claim 1, wherein the labeled peptide comprises at least SEQ ID NO: 3.

6. The method of claim 1, wherein the label molecule of the labeled peptide is located at the C-terminus, the N-terminus or at both termini.

7. The method of claim 1, wherein the labeled peptide is between about 13 to 50 amino acids in length.

8. The method of claim 1, wherein the labeled peptide comprises an epitope tag disposed at the C-terminus, the N-terminus, or at both termini.

9. The method of claim 1, wherein the sample comprises serum, plasma, cerebrospinal fluid (CSF), synovial fluid, cystic fluid, lymph fluid, ascites, pleural effusion, interstitial fluid, ocular fluids, vitreal fluid, or a combination thereof.

## Patentansprüche

1. Verfahren zur Detektion von MSDX Komplex-1 in einer Probe, wobei MSDX Komplex-1 ein Proteinkomplex umfassend Fibrinogen, Fibronektin und Fibulin-1 ist, wobei das Verfahren umfasst:
(a) Einbringen eines Anti-Peptid Antikörpers und eines markierten Peptids in die Probe, um eine Antikörper-Proben-Mischung herzustellen, wobei der MSDX Komplex-1 mit dem Anti-Peptid Antikörper um die Bindung an das markierte Peptid kompetitiert;
(b) Bereitstellen einer Kavität, die mit einem Kavitäten-Antikörper beschichtet ist;
(c) Einbringen der Antikörper-Proben-Mischung in die Kavität; und
(d) Einbringen eines Substrates in die Kavität, wobei ein Markierungsmolekül des markierten Peptids und das Substrat interagieren, um einen Signallevel bereitzustellen; wobei ein Signallevel indikativ für einen Level an MSDX Komplex-1 in der Probe ist.

2. Verfahren gemäß Anspruch 1, wobei das Markierungsmolekül Biotin ist.

3. Verfahren gemäß Anspruch 1, wobei das markierte Peptid mindestens die SEQ ID NO:1 umfasst.

4. Verfahren gemäß Anspruch 1, wobei das markierte Peptid mindestens die SEQ ID NO:2 umfasst.

5. Verfahren gemäß Anspruch 1, wobei das markierte Peptid mindestens die SEQ ID NO:3 umfasst.

6. Verfahren gemäß Anspruch 1, wobei das Markierungsmolekül des markierten Peptids am C-Terminus, am N-Terminus oder an beiden Termini lokalisiert ist.

7. Verfahren gemäß Anspruch 1, wobei das markierte Peptid eine Länge von etwa 13 bis 50 Aminosäuren aufweist.

8. Verfahren gemäß Anspruch 1, wobei das markierte Peptid über einen Epitop-Tag am C-Terminus, am N-Terminus oder an beiden Termini verfügt.

9. Verfahren gemäß Anspruch 1, wobei die Probe Serum, Plasma, Rückenmarksflüssigkeit (CSF), Synovialflüssigkeit, Cystenflüssigkeit, Lymphflüssigkeit, Aszites, Pleuraerguss, interstitielle Flüssigkeit, Okularflüssigkeit, Glaskörperflüssigkeit oder eine Kombination davon umfasst.

## Revendications

1. Procédé de détection du complexe 1 de MSDX, le complexe 1 de MSDX étant un complexe protéique comprenant du fibrinogène, de la fibronectine et de la fibuline-1 dans un échantillon, le procédé consistant à :
(a) introduire à l'échantillon un anticorps anti-peptide et un peptide marqué pour créer un mélange anticorps-échantillon, où le complexe 1 de MSDX entre en compétition avec l'anticorps anti-peptide pour se lier au peptide marqué ;
(b) fournir un puits revêtu d'un anticorps de puits ;
(c) introduire le mélange anticorps-échantillon au puits ; et
(d) introduire un substrat au puits, où une molécule de marquage du peptide marqué et le substrat interagissent pour fournir un niveau d'un signal ; où un niveau de signal indique un niveau du complexe 1 de MSDX dans l'échantillon.

2. Procédé de la revendication 1, dans lequel la molécule de marquage est la biotine.

3. Procédé de la revendication 1, dans lequel le peptide marqué comprend au moins la séquence SEQ ID NO: 1.

4. Procédé de la revendication 1, dans lequel le peptide marqué comprend au moins la séquence SEQ ID NO: 2.

5. Procédé de la revendication 1, dans lequel le peptide marqué comprend au moins la séquence SEQ ID NO: 3.

6. Procédé de la revendication 1, dans lequel la molécule de marquage du peptide marqué est située au niveau de l'extrémité C-terminale, de l'extrémité N-terminale ou au niveau des deux extrémités terminales.

7. Procédé de la revendication 1, dans lequel le peptide marqué a une longueur comprise entre environ 13 et 50 acides aminés.

8. Procédé de la revendication 1, dans lequel le peptide marqué comprend un marqueur épitopique disposé au niveau de l'extrémité C-terminale, de l'extrémité N-terminale ou au niveau des deux extrémités terminales.

9. Procédé de la revendication 1, dans lequel l'échantillon comprend du sérum, du plasma, du liquide céphalorachidien (LCR), un liquide synovial, un fluide kystique, un liquide lymphatique, une ascite, un épanchement pleural, un liquide interstitiel, des fluides oculaires, un liquide vitréen, ou une combinaison de ceux-ci.
